# EUROPÄISCHE PATENTSCHRIFT

(11) **EP 0 607 775 B1**
(45) Veröffentlichungstag und Bekanntmachung des Hinweises auf die Patenterteilung: **09.12.1998**
(21) Anmeldenummer: 94100013.5
(22) Anmeldetag: 03.01.1994
(51) Int. Cl.: A61K 31/42

(54) **Verwendung von Leflunomid zur Hemmung von Interleukin 1 beta**
Use of leflunomid for the inhibition of interleukin 1 beta
Utilisation de leflunomid pour inhiber interleukin 1 bêta

(30) Priorität: 08.01.1993 DE 4300277
(43) Veröffentlichungstag der Anmeldung: 27.07.1994
(73) Patentinhaber: HOECHST AKTIENGESELLSCHAFT, 65929 Frankfurt am Main (DE)
(72) Erfinder: Weithmann, Klaus Ulrich, Dr., D-65719 Hofheim (DE); Bartlett, Robert Ryder, Dr., D-64291 Darmstadt (DE)

(56) Entgegenhaltungen:
- EP-A- 0 013 376
- WO-A-91/17748
- WO-A-92/02822
- WO-A-92/16226
- IMMUNOPHARMACOLOGY Bd. 23, Nr. 2 , 1992 Seiten 105 - 116 T. GLANT ET AL. 'Immunomodulation of proteoglycan-induced progressive polyarthritis by leflunomide'
- IMMUNOBIOLOGY Bd. 186, Nr. 1-2 , 1992 Seite 113 T. ZIELINSKI ET AL. 'Effects of leflunomide (HWA 486) on cell cycle distribution and expression of lymphyocyte activation markers'
- AGENTS AND ACTIONS Bd. 32, Nr. 1-2 , 1991 Seiten 10 - 21 R. BARTLETT ET AL. 'Leflunomide (HWA 486), a novel immunomodulating compound for the treatment of autoimmune disorders and reactions leading to transplantation rejection'
- AGENTS AND ACTIONS Bd. 38, Nr. SPEC , 1993 Seiten C80 - C82 T. ZIELINSKI ET AL. 'Effects of leflunomide (HWA 486) on expression of lymphocyte activation markers'
- FEBS LETTERS Bd. 334, Nr. 2 , November 1993 Seiten 161 - 164 T. MATTAR ET AL. 'Inhibition of the epidermal growth factor receptor tyrosine kinase acitivty by leflunomide'

## Beschreibung

Leflunomid (s. Formel, N-(4-Trifluormethylphenyl)-5-methyl-isoxazol-4-carboxamid) ist als chemische Verbindung bereits bekannt (EP 0013376, EP0217206, US 4351841, US 4965276).

Neben den bereits offenbarten antiphlogistischen Effekten bewirkt diese Substanz auch immunomodulatorische Wirkungen die sie zur Behandlung von Autoimmunerkrankungen und Transplantatabstossungsreaktionen befähigen. Es ist auch schon bekannt, daß dieser Metabolit mit der Bezeichnung N-(4-Trifluormethylphenyl)-2-cyano-3-hydroxy-crotonsäureamid (s. Formel) für die heilenden Wirkungen von Leflunomid verantwortlich ist.

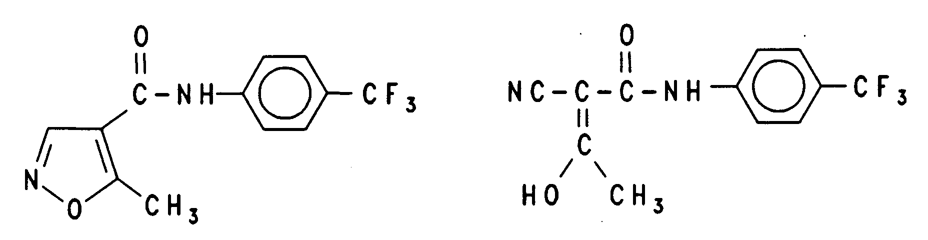

In Übereinstimmung mit dieser Erkenntnis können die oben zitierten pharmakologischen Wirkungen von Leflunomid auch durch Applikation dieses genannten Metaboliten erhalten werden (Bartlett et al, Agents and Actions, 32 (1991) 10-21).

Auch in Axton et al, J.Chem.Soc.Perkin Trans. 1 (1992) 2203ff ist beschrieben worden, daß nicht Leflunomid das aktive Prinzip darstellt, sondern daß dieser primäre Metabolit die biologischen Wirkungen entfaltet.

Sowohl in der Literatur (Bartlett et al, Agents and Actions, 32 (1991) 10-21), als auch in eigenen Experimenten, konnte gezeigt werden, daß die im folgenden näher beschriebenen therapeutischen Wirkungen durch Applikation des Leflunomid-Metaboliten nicht erhalten werden können. Erfindungsgemäß wurde nämlich gefunden, daß Leflunomid einen starken inhibitorischen Einfluß auf die Synthese bzw. Freisetzung von Zytokinen aus menschlichen Blutzellen besitzt, wohingegen der Leflunomid-Metabolit diese vorteilhafte Wirkung nicht aufweist.

Unter den erfindungsgemäß angewandten experimentellen Bedingungen findet keine nennenswerte Metabolisierung des Leflunomid statt, und die inhibierende Wirkung ist ausschließlich der Substanz Leflunomid zuzuschreiben.

Bei den Zytokinen handelt es sich um eine Klasse verschieden-artiger, biologisch hochpotenter Peptide, deren Strukturen bereits bekannt sind. Es ist ebenfalls schon bekannt, daß sie endogen als Transmittersubstanzen induziert und synthetisiert werden.

Die Unterdrückung von Zytokinen im menschlichen oder tierischen Körper ist deshalb von großer medizinischer Bedeutung, weil überhöhte Spiegel dieser Zytokine zum Auftreten bzw. Ausbrechen zahlreicher Krankheiten führen können.

Zwar könnte zur Behandlung solcher Krankheiten auch ein Medikament eingesetzt werden, das die unerwünschte Wirkung des gegebenenfalls bereits vorhandenen Zytokines auf Organ-, Zell-, Gewebe- und Rezeptorsysteme des Körpers verhindert; es ist nun jedoch ein weiterer signifikanter Vorteil der vorliegenden Erfindung, daß durch Einsatz von Leflunomid bereits die Synthese bzw. Freisetzung des Zytokines verhindert wird, so daß dieses gar nicht erst entsteht, und somit die Entstehung der Krankheit bereits in einer sehr frühen Phase verhindert werden kann.

Die vorliegende Erfindung betrifft die Verwendung von Leflunomid zur Herstellung eines Arzneimittels zur Vorbeugung und Behandlung von Krankheiten des menschlichen und tierischen Körpers, die durch das Zytokin mit der Bezeichnung Interleukin 1 beta (IL1β) bewirkt werden, mit der Maßgabe, daß die Krankheiten rheumatische Arthritis und Leukümie ausgeschlossen sind.

IL1ß und seine krankheitsverursachenden Wirkungen sind ausführlich in Ibelgaufts, Lexikon Zytokine, Medikon Verlag, München 1992, und in der dort zitierten Literatur beschrieben.

Aber auch z. B. in WO 92/02822 und WO 91/155577, sowie in Thornberry et al, Nature 356 (1992) 768-774, Cerretti et al, Science 256 (1992) 97-100 und Eastgate, Duff et al, Lancet 2 (1988) 706-709 wird auf die unerwünschten Wirkungen von IL1ß hingewiesen. Über die zentrale Rolle von IL1β beim septischen Schock, bei Leukämie und Hepatitis wurde in Tanaka et al, An. Letts 21 (1988) 169-181 berichtet.

Darüber hinaus hat IL1β ein breites biologisches pathogenes Aktivitätsspektrum bei z. B. Muskelabbau, HIV-Infektion und Hirnstoffwechselerkrankungen wie die Alzheimersche Krankheit.

Die Synthese von IL1β kann in zahlreichen Zellen wie Makrophagen, Fibroblasten, Endothelzellen und Lymphozyten erfolgen; besonders aktiv ist sie in peripheren Monozyten. So wird verständlich, daß IL1β eine zentrale Stellung bei besonders schwerwiegenden Krankheiten, deren Behandlung heute gar nicht oder nur unzureichend möglich ist, einnimmt. Auch deshalb kommt der aufgefundenen Wirkung von Leflunomid eine große Bedeutung zu.

Die vorliegende Erfindung betrifft ferner die Verwendung von Leflunomid zur Vorbeugung und Behandlung von Krankheiten mit erhöhter Knorpelresorption, Gehirnhautentzündungen, Mycobakterieller Infektionen, Thrombosen, arteriosklerotische Ablagerungen, erhöhtem Fettspiegel oder Gelenkzerstörung (Mustafa et al., J. Pediat., 115 (1989) Seite 1274 ff.; Kindler et al., Cell, 56 (1989) seite 731 ff.; Joly et al., Circ. Res., 71 (1992) Seite 331 ff.; Ku et al., JBC, 267 (1992) Seite 14183; Chin et al., Arthritis and Rheumatism, 34 (1991) Seite 314 ff.).

Zur Behandlung dieser Krankheiten können insbesondere auch Arneimittelformen und galenische Zubereitungen von Leflunomid, die auf üblichem Wege hergestellt worden sind, Verwendung finden.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit-oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyätthylenglykole und Lösungsmittel, wie etwa sterile Wasser und ein oder mehrwertige Alkohole, z. B. Glycerin, genannt.

In der Humanmedizin werden Dosiseinheiten mit 3 bis 5 mg, vorzugsweise 10, 25 oder 50 mg pro Patient (70 kg Körpergewicht) verabfolgt. Falls medizinisch erforderlich kann die Dosiseinheit bis auf 100, 200 oder 500 mg pro Patient gesteigert werden. Die Dosierung kann einmal täglich bis einmal wöchentlich, vorzugsweise bis zu drei oder viermal täglich erfolgen. Die Applikation kann oral, peritoneal, intravenös, intraartikulär oder transdermal auf übliche Weise erfolgen. Aus diesen Angaben lassen sich auch leicht die wirksamen veterinärmedizinischen Applikationen berechnen.

Schließlich kann Leflunomid bei der Herstellung der vorgenannten galensichen Zubereitungsformen auch zusammen mit anderen geeigneten Wirkstoffen, beispielsweise Antiuricopathika, Thrombocytenaggregationshemmern, Analgetika und anderen steroidalen oder nicht-steroidalen Antiphlogistika, formuliert werden.

Experimentell konnten die Wirkungen von Leflunomid an einer isolierten Blutzellfraktion (mononukleare Zellen) nachgewiesen werden, die insbesondere keine nennenswerte Metabolisierung des Leflunomids zu seinem Metaboliten bewirkte.

### Beispiel 1

Die Anreicherung der mononuklearen Zellen aus frisch gewonnenem menschlichen Zitratblut erfolgte nach bekannten Standardverfahren (s. Tiku et al, J. Immunol. 136/10 (1986) 3677):

10 ml frisch hergestelltes menschliches Zitratblut wurden vorsichtig mit 15ml Lymphoprep^{(R)} (Molter GmbH, Heidelberg) unterschichtet und dann bei 400xg 40 min bei 20°C zentrifugiert. Die sich in der Phasengrenze als weißer Ring abzeichnende Zellfraktion wurde mit Hilfe einer Spritze herausgezogen, 1:1 (v/v) mit PM-16-Puffer (Fa. Serva Feinbiochimica GmbH & Co KG, Heidelberg) verdünnt und wie oben für 10 min zentrifugiert. Der Überstand wurde mit 10 ml RPMI 1640-Puffer (Gibco, Berlin), dem vorher 300 mg/l L-Glutamin zugesetzt worden waren, gewaschen. Die gewaschene Zellfraktion wurde in 1 ml RPMI 1640, dem vorher 300 mg /l L-Glutamin, 25 mmol/l HEPES (Gibco, Berlin), 0,1 g/ml Streptomyzin und 0,1 g/ml Penizyllin zugesetzt worden waren, aufgenommen. Mit Hilfe eines Zellzählers (Typ IT , Fa. Coulter Diagnostics, Krefeld) wurde die Zellsuspension, die aus ca. 90 % Lymphozyten und 10 % Monozyten besteht, auf etwa 5 Millionen Zellen/ml eingestellt. Die Zellviabilität wurde vor und nach den Inhibierungsexperimenten mit Hilfe der bekannten Laktatdehydrogenase-Methode überprüft. Eine Änderung der Viabilität wurde dabei nicht festgestellt.

Die Synthese und Freisetzung von zellulärem IL1β wurde induziert, indem zu 0,48 ml der oben beschriebenen Zellfraktion eine Lösung von 500 ng Lipopolysacharid (Salmonella abortus equi, Sigma GmbH, Deisenhofen) in 0,01 ml Dimethylsulfoxid/Wasser (1:10, v/v) gegeben wurde. Gleichzeitig wurde die Zellfraktion mit einer Lösung von Leflunomid oder von Leflunomid-Metabolit in 0,01 ml Dimethylsulfoxid (jeweilige Endkonzentration siehe Tab. 1) versetzt, und das Gemisch wurde für 20 h bei 37°C in einem handelsüblichen Inkubator belassen. Nach dem Abkühlen 0°C wurden die Proben 1 min in einer Tischzentrifuge zentrifugiert, und jeweils 0,025-ml-Aliquote des Überstandes wurden mit Hilfe des "Sandwich" Enzym-Immuno--Testkits (Fa. Biermann GmbH, Bad Nauheim) nach Herstellervorschrift spezifisch auf ihren IL1β-Gehalt untersucht. Die Kontrollwerte wurden ohne Zusatz von Leflunomid oder Metabolit bestimmt und auf 100 % gesetzt. Insbesondere wurde durch entsprechende Vergleichsmessungen ein etwaiger Einfluß von Dimethylsulfoxid auf den IL1β-Spiegel ausgeschlossen.

Weiterhin wurden Aliquote des Leflunomid enthaltenden Testansatzes zeitabhängig entnommen und mit Hilfe der der Hochdruckflüssigchromatographie (C-18-Säule 3.9x150 mm, Waters GmbH, Eschborn, Laufmittel: 600 ml Methanol/350 ml Wasser/ 50 ml Tetrahydrofuran/ 1 ml Phosphorsäure; Flußrate 0,7 ml/min bei 2000 pounds per ssquare inch (Psi); Detektion im ultravioletten Bereich bei 273 nm) auf ihren Gehalt an Leflunomid und Leflunomid-Metabolit überprüft. Es zeigte sich, daß Leflunomid unter den angewandten Bedingungen nur sehr langsam, mit einer Halbwertszeit von ca. 10 Stunden, metabolisiert wird.

**Tabelle 1**

| Prüfsubstanz | Konzentration Versuche mmol/l | IL1β im Überstand +/-Standard-Abweichung | Anzahl n = |
|---|---|---|---|
| Leflunomid | | | |
| | 0,1 | 17+/-4 | 6 |
| | 0,05 | 27+/-6 | 3 |
| | 0,01 | 70+/-8 | 6 |
| | 0,005 | 68 | 2 |
| | 0,0001 | 86+/-11 | 4 |

| Leflunomid-Metabolit | | | |
|---|---|---|---|
| | 0,1 | 99+/-12 | 3 |
| | 0,01 | 100+/-3 | 4 |
| ohne | 0 | 100 | |

Die o.g. Experimente zeigen, daß der Leflunomid-Metabolit praktisch keinen Einfluß auf den IL1β-Spiegel bewirkt, während der IL1β-Spiegel nach Gabe von Leflunomid deutlich gesenkt wird.

### Beispiel 2

### Herstellung von N-(4-Trifluormethylphenyl)-5-methyl-isoxazol-4-carboxamid

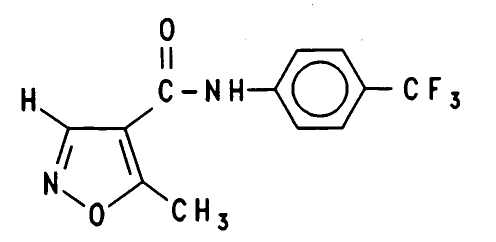

Eine Lösung von 0,05 Mol 4-Methylisoxazol-4-carbonsäurechlorid (7,3 g) in 20 ml Acetonitril werden tropfenweise bei Raumtemperatur in eine Lösung von 0,1 Mol 4-Trifluormethylanilin (16,1 g) in 150 ml Acetonitril gegeben. Nach 20 min Rühren wird das ausgefallene 4-Trifluormethylanilin-hydrochlorid abgesaugt, zweimal mit je 20 ml Acetonitril gewaschen und die vereingten Filtrate unter vermindertem Druck eingeengt. Ausbeute: 12,8 g weißes, kristallines N-(4-Trifluormethylphenyl)-5-methyl-isoxazol-4-carboxamid (Leflunomid).

### Beispiel 3

### Akute Toxizität nach intraperitonealer Verabreichung

Die akute Toxizität nach intraperitonealer Verabreichung der Testsubstanz wurde mit NMRI-Mäusen (20 bis 25 g) und SD-Ratten (120 bis 195 g) durchgeführt. Die Testsubstanz wurde in einer 1 %igen Natrium-Carboxymethylcellulose-Lösung suspendiert. Die verschiedenen Dosierungen der Testsubstanz wurden den Mäusen in einem Volumen von 10 ml/kg Körpergewicht und den Ratten in einem Volumen von 5 ml/kg Körpergewicht verabreicht. Pro Dosierung wurden 10 Tiere verwendet. Nach 3 Wochen wurde die akute Toxizität nach der Methode von Litchfield und Wilcoxon bestimmt. Die Ergebnisse sind in der Tabelle 2 zusammengefaßt.

**Tabelle 2**

| | Leflunomid akute Toxizität intraperitoneal LD₅₀ (mg/kg) |
|---|---|
| NMRI-Maus | 185 (163 - 210) |
| SD-Ratte | 170 (153 - 189) |

## Patentansprüche

1. Verwendung von N-(4-Trifluormethylphenyl)-5-methyl-isoxazol-4-carboxamid zur Herstellung eines Arzneimittels zur Vorbeugung und Behandlung von Krankheiten, die durch Interleukin 1 beta bewirkt werden, mit der Maßgabe, daß die Krankheiten rheumatische Arthritis und Leukämie ausgeschlossen sind.

2. Verwendung gemäß Anspruch 1, zur Vorbeugung und Behandlung von Krankheiten wie erhöhter Knorpelresorption, Hepatitis, HIV-Infektion, Hirnstoffwechselerkrankungen wie Alzheimersche Krankheit, Muskelabbau, Gehirnhautentzündung, Mycobakteriellen Infektionen, Thrombosen, arteriosklerotische Ablagerungen, erhöhtem Fettspiegel oder Gelenkzerstörung.

## Claims

1. The use of N-(4-trifluoromethylphenyl)-5-methylisoxazole-4-carboxamide for preparing a pharmaceutical for preventing and treating disorders which are caused by interleukin 1 beta, with the proviso that the disorders rheumatoid arthritis and leukemia are excluded.

2. The use as claimed in claim 1, for preventing and treating disorders such as increased cartilage resorption, hepatitis, HIV infection, disorders of brain metabolism such as Alzheimer's disease, muscle breakdown, meningitis, mycobacterial infections, thromboses, arteriosclerotic depositions, an elevated fat level or joint destruction.

## Revendications

1. Utilisation du N-(4-trifluorométhylphényl)-5-méthylisoxazole-4-carboxamide pour la fabrication d'un médicament destiné à la prévention et au traitement de maladies qui sont provoquées par l'interleukine 1β, étant entendu que sont exclues les maladies polyarthrite rhumatoïde et leucémie.

2. Utilisation selon la revendication 1, pour la prévention et le traitement de maladies telles que la résorption cartilagineuse accrue, l'hépatite, l'infection à VIH, des maladies du métabolisme cérébral telles que la maladie d'Alzheimer, la dégradation musculaire, la méningite, les infections mycobactériennes, les thromboses, les dépôts artérioscléreux, l'hyperlipidémie ou la destruction articulaire.
